Europäisches Patentamt

European Patent Office

Office européen des brevets

(11) Numéro de publication: **0 176 421**
**B1**

(12) **FASCICULE DE BREVET EUROPEEN**

(45) Date de publication du fascicule du brevet:
**12.04.89**

(51) Int. Cl.⁴: **A 61 F 2/36**

(21) Numéro de dépôt: **85401752.2**

(22) Date de dépôt: **09.09.85**

(54) **Implant pour prothèse coxo-fémorale.**

(30) Priorité: **10.09.84 FR 8413871**

(43) Date de publication de la demande:
**02.04.86 Bulletin 86/14**

(45) Mention de la délivrance du brevet:
**12.04.89 Bulletin 89/15**

(84) Etats contractants désignés:
**CH DE FR GB IT LI**

(56) Documents cités:
**CH-A- 599 784**
**DE-A- 2 627 569**
**DE-A- 3 003 050**
**FR-A- 80 495**
**FR-A- 2 299 012**
**FR-A- 2 315 902**
**FR-A- 2 419 717**
**FR-A- 2 425 843**
**FR-A- 2 464 695**
**FR-A- 2 549 717**
**US-A- 3 918 441**
**US-A- 4 068 324**

(73) Titulaire: **Fournier, Jacques André, 2 Boulevard Edouard Lachaud, F-19100 Brive (FR)**

(72) Inventeur: **Fournier, Jacques André, 2 Boulevard Edouard Lachaud, F-19100 Brive (FR)**

(74) Mandataire: **Dupuy, René Gaston, Cabinet DUPUY & LOYER 14 rue La Fayette, F-75009 Paris (FR)**

ACTORUM AG

## Description

L'invention se rapporte à une prothèse coxofémorale comportant un implant destiné à être engagé dans la cavité médullaire du fémur en vue du remplacement des deux parties de l'articulation défaillante pour des raisons pathologiques ou pour cause d'accident.

Il est bien connu que le gros problème à résoudre réside alors dans l'ancrage de la prothèse dans le fémur.

Depuis un peu plus de trente ans, deux techniques d'ancrage ont été utilisées simultanément:
– l'ancrage à l'aide d'un ciment chirurgical par exemple à base de métacrylate de méthyle.
– L'ancrage sans ciment selon lequel le blocage est obtenu par ostéogenèse au contact de l'implant dans la métaphyse fémorale.

Il est aisé de comprendre que sur le plan de la transmission des efforts ces deux techniques sont fondamentalement différentes, même si elles mettent en jeu des structures présentant entre elles certaines ressemblances.

La présente invention vise une prothèse coxofémorale du type n'utilisant pas de ciment.

Il existe de nombreux brevets se rapportant à ce type de prothèse.

On y trouve la description d'implants souvent dénommés «broches» sensiblement incurvés pour suivre sensiblement l'axe du fémur dans lequel ils sont implantés (par exemple. FR-A 2 242 065, FR-A 2 194 123, FR 2 528 307, FR-A 2 299 012, FR-A 80 495, DE-A 3 003 050).

La queue de l'implant est soit lisse pour blocage par du ciment (FR-A 2 299 012), soit partiellement lisse (FR 2 528 307) soit grenue (FR-A 2 194 123) généralement de section circulaire, mais pouvant être aussi de section cruciforme (FR-A 80 495) ou polygonales (FR-A 2 528 307, FR-A 2 419 717). On trouve aussi des queues pourvues d'une pluralité de saillies annulaires à la manière de celle d'un tire-fond (FR-A 2 242 065, FR-A 2 295 730), saillies qui sont normales par rapport à l'axe de l'implant et destinées dans l'esprit de leur inventeur à être perpendiculaires à la force de mise en charge.

On peut citer également les prothèses à implant vissé dans la cavité médullaire.

Il faut remarquer, dans toutes celles qui viennent d'être décrites, que le blocage s'effectue par application d'une platine perpendiculaire à l'axe de l'implant, sur la partie recepée du grand trochanter ou sur la cassure cervico-trochanterienne comme dans le cas du FR-A 2 528 307.

Les observations cliniques ont montré que de telles prothèses à queue légèrement courbée, à platine et a saillies perpendiculaires à l'axe de l'implant n'étaient pas exemptes d'inconvénients et ne répondaient nullement aux espoirs de leur inventeur.

On peut citer une défectuosité de l'assise, à plus ou moins longue échéance, et une mauvaise répartition de la pression:
le profil incurvé lui-même peut produire
– un mouvement de bascule en varus d'où excès de pression côté interne, sur le plan frontal et quasi inexistance de pression sur le plan sagittal,
– une possibilité de fracture de la queue ou de bascule interne avec réaction corticale au niveau de celle-ci.

La mauvaise orientation des saillies peut conduire à des microfractures des néoformations osseuses susceptible de faire migrer l'implant, soit par enfoncement, soit en le libérant avec pour conséquence une prothèse douloureuse.

Il faut noter en outre que la présence de ces saillies transversales rend très difficile une extraction, en cas d'intervention ultérieure.

DE-A 2 627 569 décrit un implant pour prothèse coxo-fémorale comportant une queue sensiblement rectiligne, légèrement conique, devant être engagée dans la cavité médullaire du fémur et un corps portant le tenon ou col de la rotule d'articulation, ledit implant comportant entre ledit corps et ladite queue une saillie inclinée, prédominante sur les faces antérieure et postérieure de la queue, inclinée depuis un bord interne du côté de la paroi corticale interne vers un bord externe du côté de la paroi corticale externe, destinée à venir appliquer contre la surface de la coupe du fémur après sa résection au niveau du grand trochanter.

Il s'agit d'un implant destiné à être bloqué avec du ciment qui comporte en outre une platine cervicale sensiblement perpendiculaire à l'axe de l'implant ce qui provoque les inconvénients mentionnés précédemment.

L'implant selon l'invention est caractérisé en ce que ladite saillie est de forme lancéolée ou en amande, s'élargissant depuis le bord interne jusqu'à mi-longueur puis se rétrécissant jusqu'à former une pointe au niveau du bord externe, que ladite saillie est surmontée de plusieurs autres saillies de même forme, lesdites saillies étant inclinées sensiblement de 130° par rapport à l'axe longitudinal de la queue.

Les autres caractéristiques de l'invention font l'objet des sous-revendications.

Il est aisé de comprendre que l'absence de platine d'appui au niveau de la partie supérieure de la prothèse, d'une part, et la présence des saillies obliques sur les deux faces antérieure et postérieure, d'autre part, vont permettre à l'implant de trouver lui-même sa place.

En effet, au cours de la mise en place de la prothèse, ces saillies vont guider et orienter son glissement et à plus long terme, l'os spongieux qui va être entraîné et tassé contre l'intérieur de la paroi corticale va se transformer en barrettes osseuses qui vont servir de support supplémentaire et diminuer, ainsi, la pression au niveau de la paroi corticale interne.

Il faut remarquer également que ces barrettes osseuses ont la direction globale des lignes de force des parois corticales, antérieure et postérieure, du fémur normal.

Ainsi, l'appui artificiel va doubler, de la manière la plus anatomique possible, la structure naturelle de l'os. Autrement dit, l'originalité de cette prothèse est, d'une part, d'être un implant dont le glissement est contrôlé par le profil qu'il présente et, d'autre part, de se bloquer dans la bonne position, en raison de sa forme en tulipe, aussi bien dans le plan frontal que dans le plan sagittal.

Il est à noter que ce profil remplit, au mieux, l'espace disponible dans la partie haute du fémur pour se raccorder, le plus précisément possible le long des parois corticales de la métaphyse fémorale supérieure.

Enfin, selon une dernière originalité de ce profil, de façon à limiter le glissement et l'enfoncement excessif au cours de la mise en place et de l'impaction de la prothèse, le bord interne de celle-ci est plus concave que la paroi corticale fémorale correspondante. C'est ainsi que toute tentative d'impaction excessive va mettre en appui la partie interne de la prothèse qui ne pourra, ensuite, que subir une translation vers la paroi corticale externe mettant en contact les saillies lancéolées du bord externe de la prothèse avec la paroi corticale externe du fémur.

Toutes ces dispositions contribuent, bien entendu, à un blocage extrêmement puissant, en particulier, des mouvements éventuels de rotation, déjà fortement limités par la forme générale de l'extrémité supérieure de l'implant.

D'autres particularités et avantages apparaîtront à la lecture de la description et des revendications qui suivent faites en regard des dessins annexés sur lesquels:

La fig. 1 est une vue en élévation d'une prothèse coxo-fémorale comportant l'implant selon la présente invention.
La fig. 2 étant une vue du corps côté col.

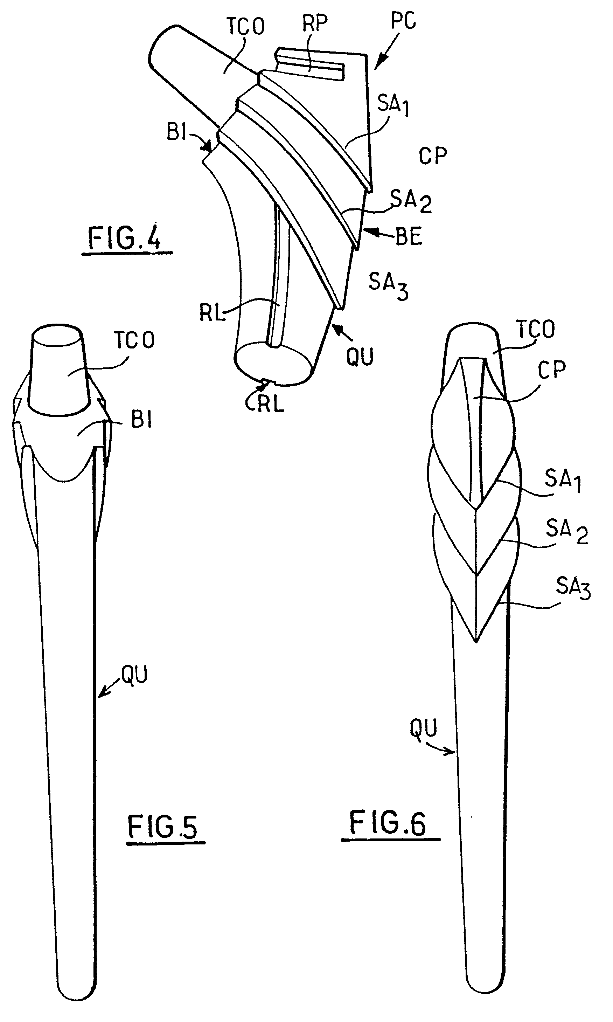

La fig. 3 étant une coupe selon III-III de la fig. 1
La fig. 4 étant une vue en perspective du corps
Les fig. 5 et 6 étant respectivement des vues de profil de l'implant côté interne et côté externe.

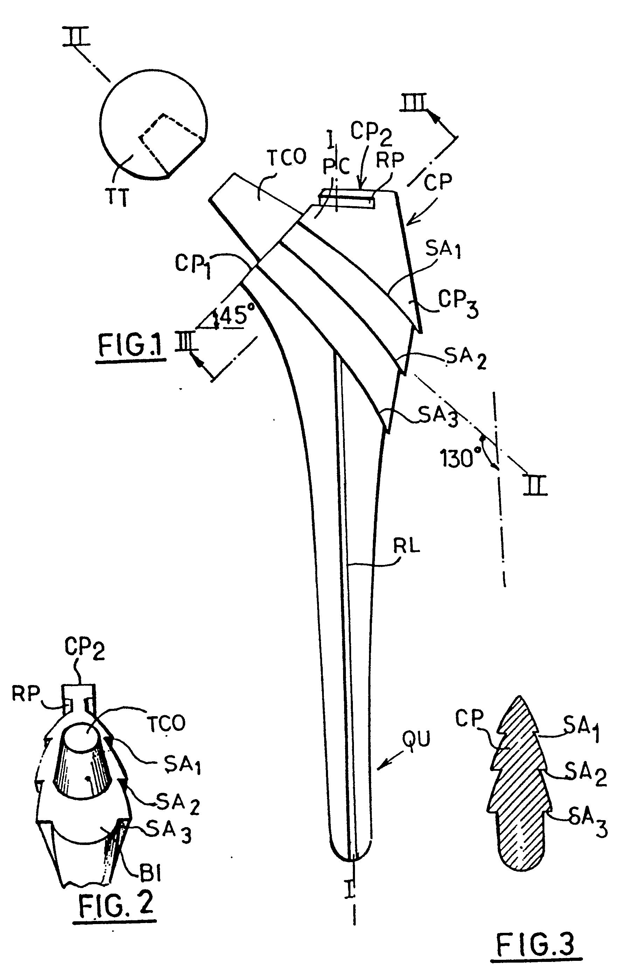

Comme on le voit sur la figure 1, la prothèse en cause comporte l'implant proprement dit constitué par la queue QU et le corps CP, et le col formé d'une part du tenon conique TCO solidaire dudit corps et d'autre part, de la tête sphérique TT à emmanchement conique complémentaire (avantageusement cône mors de coincement dont la génératrice forme avec l'axe un angle compris entre 4 et 7°).

La queue QU est à axe rectiligne I-I par rapport auquel l'axe II-II du col fait un angle de sensiblement 130°.

La queue QU comporte des saignées longitudinales RL rainées dans au moins deux faces latérales et destinées à permettre la remontée de la moelle osseuse chassée par la partie conique, au fur et à mesure de son enfoncement dans le canal médullaire. Ces saignées se prolongent jusqu'à la saillie inférieure de manière à contenir le plus possible de la moelle, évitant ainsi la création d'une hyperpression dans ce canal et par conséquent les risques d'embolie graisseuse.

Les FR-A 80 495 et FR-A 2 419 717 bien que décrivant une queue partiellement rainée (ou cruciforme) ne résolvent pas ce problème en ne faisant que déplacer la zone où se manifestera cette hyperpression.

Le corps CP s'élargit de bas en haut dans les deux plans: frontal et sagittal.

Le corps CP est limité du côté interne (col) par une surface plane $CP_1$ perpendiculaire à l'axe II-II et sur le dessus par une autre surface plane $CP_2$ perpendiculaire à l'axe I-I.

Le corps CP du côté interne BI prolonge la queue QU sans décrochement, alors qu'il est raccordé à celle-ci latéralement sur les faces avant et arrière par une pluralité de saillies $SA_1$, $SA_2$, $SA_3$ ménageant entre elles des redans à flancs inclinés symétriquement au plan axial médian de l'implant.

Avantageusement, ces saillies $SA_1$, $SA_2$, $SA_3$ ont une forme lancéolée ou en amande comme il sera décrit plus loin. Au moins, la saillie la plus inférieure $SA_3$, du fait de son inclinaison et de sa forme constitue une sorte de patin ou de sabot propice à favoriser le glissement en direction de la paroi corticale externe et par là la mise en place de l'implant.

Le bord externe BE présente une partie haute sensiblement verticale $CP_3$ et une partie basse dont le profil se recourbe pour rejoindre le profil externe de la queue QU déterminant ainsi un évasement vers le haut et vers l'extérieur.

C'est à ce bord qu'aboutissent les sailles $SA_1$, $SA_2$ et $SA_3$ qui y forment au niveau de la paroi corticale externe du fémur des ergots de fixation constitués par la jonction des faces antérieure et postérieure des saillies.

Celles-ci ont des reliefs symétriques par rapport au plan médian de l'implant, elles sont courbées vers le bas en partant de la surface $CP_1$ pour se diriger de façon légèrement divergente vers le bord externe BE de la prothèse où elles forment les ergots dont il vient d'être question.

Ces saillies vont en s'amincissant du bord interne haut (BI) vers le bord externe BE. Elles sont composées de deux versants: le versant supérieur en pente douce, vers le bas, le versant inférieur, abrupt perpendiculaire à l'axe de l'implant.

Comme il vient d'être dit ces versants se rejoignent au niveau du bord externe BE.

En résumé, les saillies $SA_1$, $SA_2$, $SA_3$ ont une trace géométrique sensiblement en amande, moyenne côté interne, large au centre et quasiment pointue côté externe.

A la partie supérieure du corps CP sont ménagées deux rainures RP sur les faces antérieures et postérieures permettant la préhension de l'implant par l'outil d'impaction qui servira également, et éventuellement d'extracteur.

Avantageusement la surface latérale de l'implant est grenue pour augmenter son adhérence et sa cohésion avec la matière environnante.

Au cours de l'introduction de l'implant, ces reliefs vont entraîner de l'os spongieux, qui va être tassé et qui va susciter la formation de barrettes osseuses, à l'intérieur de la métaphyse fémorale.

Cette formation produit trois conséquences:
a) meilleur appui métaphysaire, mieux réparti, sur une plus grande longueur,

b) la pression sur l'implant entraînera un glissement de celui-ci vers le bord externe, contribuant à le bloquer de manière encore plus étroite sur la paroi corticale externe du fémur,

c) associées à la forme évasée vers le haut de l'implant, les saillies mettent en place un mécanisme de blocage très amélioré par rapport aux implants existants.

Ainsi la prothèse selon la présente invention par sa conception originale améliore la répartition des pressions, diminue les contraintes habituelles des prothèses de hanche, en s'autopositionnant grâce à son profil et à ses saillies et se bloquant du fait de son relief étagé en tulipe.

Cette prothèse s'applique de manière polyvalente à la chirurgie de l'articulation fémorale (fracture, dysplasie, arthrose, arthrite).

**Revendications**

1. Implant pour prothèse coxo-fémorale comportant une queue (QU) sensiblement rectiligne, légèrement conique, devant être engagée dans la cavité médullaire du fémur et un corps (CP) portant le tenon ou col (TCO) de la rotule d'articulation (TT), ledit implant comportant entre ledit corps et ladite queue une saillie inclinée, prédominante sur les faces antérieure et postérieure de la queue, inclinée depuis un bord interne (BI) du côté de la paroi corticale interne vers un bord externe (BE) du côté de la paroi corticale externe, destinée à venir appliquer contre la surface de la coupe du fémur après sa résection au niveau du grand trochanter, caractérisé en ce que ladite saillie (SA₃) est de forme lancéolée ou en amande, s'élargissant depuis le bord interne (BI) jusqu'à mi-longueur puis se rétrécissant jusqu'a former une pointe au niveau du bord externe (BI), que ladite saillie (SA₃) est surmontée de plusieurs autres saillies de même forme (SA₂, SA₁), lesdites saillies (SA₃, SA₂, SA₁) étant inclinées sensiblement de 130° par rapport à l'axe longitudinal de la queue (QU).

2. Implant selon la revendication 1, caractérisé par le fait que lesdites saillies (SA₁, SA₂, SA₃) forment verticalement des redans à flancs inclinés symétriquement par rapport à un plan frontal passant par l'axe de la queue (QU).

3. Implant selon l'une quelconque des revendications 1 et 2, caractérisé par le fait que ladite queue (QU) comporte sur ses faces antérieure et postérieure une rainure longitudinale (RL).

4. Implant selon l'une quelconque des revendications précédentes, caractérisé par le fait que la partie supérieure (PC) du corps comporte sur ses faces antérieure et postérieure une rainure (RP) orthogonale à l'axe de la queue (QU) permettant la saisie de l'implant par un outil d'impaction.

**Claims**

1. Implant for a coxo-femoral prosthesis comprising a tail (QU) which is essentially rectilinear, slightly conical, and intended to be engaged in the medullar cavity of the femur, and a body (CP) bearing the tenon or neck (TCO) of the swivel joint (TT), the said implant comprising, between the said body and the said tail, an inclined projection, predominating on the front and rear faces of the tail, inclined from an inner edge (BI) on the side of the inner cortical wall towards an outer edge (BE) on the side of the outer cortical wall, intended to come to bear against the surface of the cutting of the femur after its resection at the level of the greater trochanter, characterized in that the said projection (SA₃) is of lanceolate form or almond-shaped, widening from the inner edge (BI) as far as the mid-length and then narrowing to form a point at the level of the outer edge (BI), in that the said projection (SA₃) is surmounted by several other projections of the same shape (SA₂, SA₁) the said projections (SA₃, SA₂, SA₁) being inclined essentially at 130° relative to the longitudinal axis of the tail (QU).

2. Implant according to Claim 1, characterized in that the said projections (SA₁, SA₂, SA₃) form, vertically, steps with flanks inclined symmetrically relative to a frontal plane passing through the axis of the tail (QU).

3. Implant according to either one of Claims 1 and 2, characterized in that the said tail (QU) comprises, on its front and rear faces, a longitudinal groove (RL).

4. Implant according to any one of the preceding claims, characterized in that the upper part (PC) of the body comprises, on its front and rear faces, a groove (RP) orthogonal to the axis of the tail (QU) and permitting the implant to be grasped by an impaction tool.

**Patentansprüche**

1. Implantat für eine Hüftgelenkprothese mit einem im wesentlichen gradlinigen, leicht konischen Anschlussstück (QU), das in den Markkanal des Oberschenkelknochens eingesteckt wird, mit einem Körper (CP), der den Zapfen oder Hals (TCO) des Drehgelenkes (TT) trägt, und mit einem geneigten Vorsprung zwischen dem Körper und dem Anschlussstück, der die vordere und hintere Seite des Anschlussstückes überragt, von einem inneren Rand (BI) an der inneren Wand des Oberschenkelhalses aus zu einem äusseren Rand (BE) an der äusseren Wand des Oberschenkelhalses geneigt ist und dazu dient, dass er sich an die Schnittfläche des Oberschenkelknochens nach dem Zurückschneiden auf die Höhe des grossen Rollhügels anlegt, dadurch gekennzeichnet, dass der Vorsprung (SA₃) lanzettförmig oder mandelförmig ausgebildet ist, der sich von dem inneren Rand (BI) bis zur halben Länge verbreitert und zu einer Spitze an dem äusseren Rand (BE) ausläuft, und dass der Vorsprung (SA₃) unterhalb mehrerer weiterer Vorsprünge (SA₂, SA₁) angeordnet ist, wobei die Vorsprünge (SA₃, SA₂, SA₁) etwa um 130° zur Längsachse des Anschlussstückes (QU) geneigt verlaufen.

2. Implantat nach Anspruch 1, dadurch gekennzeichnet, dass die Vorsprünge (SA₁, SA₂ SA₃) in vertikaler Richtung Stufenfolgen von geneigten Flanken symmetrisch zu einer vorderen Ebene bilden, die von der Achse des Anschlussstückes (QU) durchlaufen wird.

3. Implantat nach Anspruch 1 oder 2, dadurch gekennzeichnet, dass das Anschlussstück (QU) auf seiner vorderen und hinteren Seite jeweils eine Längsnut (RL) trägt.

4. Implantat nach einem der vorhergehenden Ansprüche, dadurch gekennzeichnet, dass der obere Teil (PC) des Körpers auf seiner vorderen und hinteren Seite jeweils eine horizontale Nut (RB) trägt, die rechtwinklig zur Achse des Anschlussstückes (QU) verläuft und das Ansetzen eines Schlagwerkzeuges an dem Implantat ermöglicht.

FIG.1

FIG. 2

FIG.3

TCO    RP    PC

SA₁

BI

CP

SA₂

BE

SA₃

## FIG.4

RL

QU

RL

TCO

BI

QU

## FIG.5

TCO

CP

SA₁

SA₂

SA₃

QU

## FIG.6